(19) Europäisches Patentamt
European Patent Office
Office européen des brevets

(11) Veröffentlichungsnummer : **0 039 426**
**B1**

(12) # EUROPÄISCHE PATENTSCHRIFT

(45) Veröffentlichungstag der Patentschrift : **18.01.84**

(51) Int. Cl.³ : **C 07 D285/00**, C 07 D417/12, A 01 N 43/72// C07C157/14

(21) Anmeldenummer : **81102771.3**

(22) Anmeldetag : **10.04.81**

(54) **Thiatriazindioxide und diese enthaltende herbizide Mittel.**

(30) Priorität : **02.05.80 DE 3016825**

(43) Veröffentlichungstag der Anmeldung : **11.11.81 Patentblatt 81/45**

(45) Bekanntmachung des Hinweises auf die Patenterteilung : **18.01.84 Patentblatt 84/03**

(84) Benannte Vertragsstaaten : **AT BE CH DE FR GB IT LI NL SE**

(56) Entgegenhaltungen :
**DE-A- 1 946 262**
**DE-A- 2 026 625**
**DE-A- 2 508 832**

(73) Patentinhaber : **BASF Aktiengesellschaft**
**Carl-Bosch-Strasse 38**
**D-6700 Ludwigshafen (DE)**

(72) Erfinder : **Hamprecht, Gerhard, Dr.**
**Rote-Turm-Strasse 28**
**D-6940 Weinheim (DE)**
Erfinder : **Acker, Rolf-Dieter, Dr.**
**Tuchbleiche 8**
**D-6906 Leimen (DE)**
Erfinder : **Wuerzer, Bruno, Dr.**
**Ruedigerstrasse 13**
**D-6701 Otterstadt (DE)**

**EP 0 039 426 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Thiatriazindioxide und diese enthaltende herbizide Mittel

Die vorliegende Erfindung betrifft neue 6H-1,2,4,6-Thiatriazin-1,1-dioxide, Verfahren zur Herstellung dieser Verbindungen, sowie Herbizide, welche diese Verbindungen enthalten und ihre Anwendung als Herbizide.

Es ist bekannt, daß substituierte 6H-1,2,4,6-Thiatriazin-(5)-on-1,1-dioxidderivate eine herbizide Wirkung haben (DE-OS 25 08 832).

Es wurde nun gefunden, daß substituierte 6H-1,2,4,6-Thiatriazin-1,1-dioxide der allgemeinen Formel I

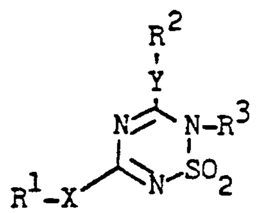

(I)

in der

$R^1$ Methyl, Ethyl, Propyl, Butyl,

$R^2$ Methyl, Ethyl, Propyl, Butyl, Pentyl, Hexyl, Allyl, Methallyl, Propargyl, Butin-1- oder -2-yl, Chlorethyl, Chlorpropyl, Chlorpropargyl, 4-Chlorbutin-1-yl-3, Chlorallyl, Methoxyethyl, Ethoxyethyl, 3-Methoxy-propyl, 3-Methoxybutyl, 1-Methoxy-butyl-2, Methylmercaptoethyl, Ethylmercaptoethyl, 3-Methylmercaptopropyl, Methylmercaptobutyl, gegebenenfalls durch Halogen, Methyl, Halogenmethyl, Halogenmethoxy substituierter Benzylrest, Rest des Propionsäure-ethylesters oder Propanonyl, wenn $R^1$ und $R^3$ = Methyl, Pentenonyl, wenn $R^1$ = Ethyl und $R^3$ = Methyl, oder gegebenenfalls durch Halogen, Methyl, Ethyl, Propyl, Butyl, Halogenmethyl, Cyan, Nitro, Methoxy, Halogenmethoxy, Methoxycarbamoyl, Methylureido, Methylmercapto, Halogenmethylmercapto, Methylsulfinyl, Methylsulfonyl, Halogenmethylsulfinyl oder Halogenmethylsulfonyl substituiertes Phenyl,

$R^3$ Methyl, Ethyl, Propyl und

X und Y Sauerstoff oder Schwefel

bedeuten, eine starke herbizide Wirkung haben und für verschiedene Kulturpflanzen verträglich sind.

Es wurde ferner gefunden, daß man die neuen Verbindungen in einfacher Weise erhält, wenn man Verbindungen der allgemeinen Formel II

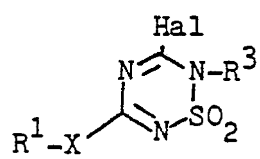

(II)

worin $R^1$, X und $R^3$ die vorgenannte Bedeutung besitzen und Hal Halogen bedeutet mit einer Verbindung der Formel III

$$H—Y—R^2 \qquad (III)$$

in der $R^2$ und Y die vorgenannte Bedeutung haben oder ihrem Alkali-, Erdalkali- oder Ammoniumsalz, gegebenenfalls in einem inerten organischen Lösungsmittel und gegebenenfalls in Anwesenheit eines Säureakzeptors bei Temperaturen zwischen − 50 und 150 °C, drucklos oder unter Druck, kontinuierlich oder diskontinuierlich umsetzt.

Verwendet man 5-Chlor-6-propyl-3-methoxy-6H-1,2,4,6-thiatriazin-1,1-dioxid und Propanol als Ausgangsmaterialien, so kann der Reaktionsablauf durch folgendes Formelschema wiedergegeben werden :

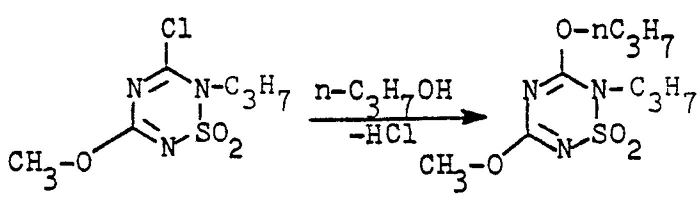


Zweckmäßig verwendet man für die Umsetzung unter den jeweiligen Reaktionsbedingungen inerte Lösungs- oder Verdünnungsmittel. Als Lösungsmittel kommen z. B. in Frage :

Halogenkohlenwasserstoffe, insbesondere Chlorkohlenwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1-2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol ; Äther, z. B. Äthylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether ; Nitrokohlenwasserstoffe wie Nitromethan, Nitroethan, Nitrobenzol, o-, m-, p-Chlornitrobenzol, o-Nitrotoluol ; Nitrile wie Acetonitril, Butyronitril, Isobutyronitril, Benzonitril, m-Chlorbenzonitril ; aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan ; Ester, z. B. Ethylacetat, Acetessigester, Isobutylacetat ; Amide, z. B. Formamid, Methylformamid, Dimethylformamid ; Ketone z. B. Aceton, Methylethylketon, gegebenenfalls auch Wasser und entsprechende Gemische. Zweckmäßig verwendet man das Lösungsmittel in einer Menge von 100 bis 2 000 Gew.-%, vorzugsweise von 200 bis 700 Gew.-%, bezogen auf Ausgangsstoff II.

Als Säureakzeptoren können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z. B. als basische Verbindungen in Frage : Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidin, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, α-Picolin, β-Picolin, γ-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Zweckmäßig verwendet man den Säureakzeptor in einem Über- oder Unterschuß von bis zu 20 %, bezogen auf den Ausgangsstoff II. Man kann jedoch auch den entstehenden Halogenwasserstoff durch Eingasen eines Inertgases, beispielsweise Stickstoff, entfernen.

Die zur Umsetzung benötigten Ausgangsstoffe III werden im allgemeinen in einem Über- oder Unterschuß von bis zu 20 %, bezogen auf den Ausgangsstoff II, eingesetzt. Man kann den Ausgangsstoff III jedoch auch direkt als Lösungsmittel verwenden. Man kann jedoch auch den Ausgangsstoff III in einem der vorgenannten Verdünnungsmittel vorlegen und dann den Ausgangsstoff II und einen Säureakzeptor, gleichzeitig oder in beliebiger Reihenfolge, über zwei getrennte Zuführungen zugeben.

Zweckmäßig wird das Verfahren zur Herstellung der neuen Verbindungen so durchgeführt, daß man den Ausgangsstoff II, gegebenenfalls in einem der vorgenannten Verdünnungsmittel vorlegt und dann den Ausgangsstoff III und einen Säureakzeptor gleichzeitig oder nacheinander zugibt. Man kann jedoch auch den Ausgangsstoff III in einem der vorgenannten Verdünnungsmittel vorlegen und dann den Ausgangstoff II und einen Säureakzeptor, gleichzeitig oder in beliebiger Reihenfolge, über zwei getrennte Zuführungen zugeben.

Die Umsetzung ist in vielen Fällen nach der Zugabe der Komponenten bereits abgeschlossen, andernfalls rührt man zu ihrer Beendigung noch 10 Minuten bis 10 Stunden bei − 50 bis 150 °C, vorzugsweise 0 bis 120 °C, insbesondere 20 bis 100 °C nach.

Verwendet man ein Inertgas zur Entfernung des Halogenwasserstoffes, so rührt man zweckmäßig 0,2 bis 10 Stunden bei 40 bis 100 °C nach.

Aus dem Reaktionsgemisch wird der Endstoff I in üblicher Weise, z. B. nach Abdestillieren von Lösungsmittel oder überschüssigem Ausgangsstoff III oder direkt durch Absaugen isoliert. Der verbleibende Rückstand wird in diesem Fall zur Entfernung saurer Verunreinigungen mit Wasser bzw. verdünntem Alkali gewaschen und getrocknet. Im Falle von mit Wasser nicht mischbaren Verdünnungsmitteln kann man auch direkt das Reaktionsgemisch mit Wasser bzw. mit verdünntem Alkali extrahieren und dann trocknen und einengen. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Chromatographie oder Destillation gereinigt werden. Die folgenden Vorschriften betreffen die Herstellung der Ausgangsverbindungen der Formel II und deren Ausgangsverbindungen.

Vorschrift 1

198 Teile (Gewichtsteile) Methylaminosulfonylchlorid und 162 Teile Triäthylamin wurden gleichzeitig

über 2 Zuführungen bei 25 bis 30 °C unter Rühren in eine Mischung von 202 Teilen N-Carbomethoxy-O-methylisoharnstoff in 1 570 Teilen Acetonitril eingeführt. Nach 3 Stunden Rühren bei 25 °C wurde vom ausgefallenen Hydrochlorid abgesaugt und das Filtrat im Vakuum engeengt. Der Rückstand wurde in 1 500 Teilen 1,2-Dichlorethan gelöst, einmal mit Wasser und zweimal mit 0,5 normaler Salzsäure extrahiert. Nach dem Trocknen über Magnesiumsulfat und Einengen im Vakuum wurden 257 Teile N-Carbomethoxy-N'-methylsulfamoyl-O-methyl-harnstoff mit $n_D^{25}$ 1,485 l erhalten.

Hiervon wurden 96 Teile in 235 Teilen Methanol (absolut trocken) gelöst, mit 153,5 Teilen Natriummethylat (30 Gewichtsprozent) versetzt und 3 Stunden unter Rückfluß gerührt. Nach dem Einengen im Vakuum wurde der Rückstand in Wasser gelöst, einmal mit Äther extrahiert und mit verdünnter Schwefelsäure angesäuert. Nach dem Absaugen, Waschen mit Wasser und Trocknen wurden 68 Teile ( = 82,5 % der Theorie) 6-Methyl-3-methoxy-6H-1,2,4,6-thiatriazin(5)-on-1,1-dioxid mit Fp. 198 bis 202 °C erhalten.

Vorschrift 2

140 Teile N-Carboxymethyl-N'-methylsulfamoyl-O-methyl-harnstoff wurden in einer Mischung von 79,5 Teilen Natriumcarbonat in 450 Teilen Wasser und 31 Volumenteilen 2 n Natronlauge 10 Minuten bei 45 °C gerührt. Das Reaktionsgemisch wurde abgekühlt, mit Äther extrahiert und langsam in eine Mischung von 78 Teilen konzentrierte Schwefelsäure in 150 Teilen Eiswasser eingerührt. Nach dem Absaugen, Waschen mit Wasser und Trocknen wurden 81 Teile 6-Methyl-3-methoxy-6H-1,2,4,6-thiatriazin(5)-on-1,1-dioxid (68 % der Theorie) mit Fp. 195 bis 199 °C erhalten.

Vorschrift 3

215 Teile 6-Methyl-3-methoxy-6H-1,2,4,6-thiatriazin(5)on-1,1-dioxid werden bei Raumtemperatur in eine Mischung von 275 Teilen Phosphorpentachlorid in 1 480 Teilen Phosphoroxychlorid unter Rühren eingeführt und innerhalb 30 Minuten auf 110 °C erhitzt. Nach 4 Stunden Rühren unter Rückfluß wurde das Reaktionsgemisch im Vakuum eingeengt, wobei 235 Teile (99,6 % der Theorie) 5-Chlor-6-methyl-3-methoxy-6H-1,2,4,6-thiatriazin-1,1-dioxid mit Fp. 77-83 °C erhalten wurden.

Vorschrift 4

154 Teile Phosphorpentachlorid wurden innerhalb 2 Minuten unter Rühren bei 25 °C zu einer Mischung von 128 Teilen 6-Ethyl-3-methoxy-6H-1,2,4,6-thiatriazin(5) on-1,1-dioxid in 840 Teilen Phosphoroxychlorid gegeben. Das Reaktionsgemisch wurde 5 1/2 Stunden unter Rückfluß gerührt und dann im Vakuum eingeengt. Das verbleibende Öl wurde in 300 Teilen 1,2-Dichlorethan aufgenommen und über neutrales Aluminiumoxid (Aktivität I) chromatographiert. Nach dem Einengen wurden 127,5 Teile (91 % der Theorie) 5-Chlor-6-ethyl-3-methoxy-6H-1,2,4,6-thiatriazin-1,1-dioxid mit Fp. 63-70 °C erhalten.

Vorschrift 5

50 Teile Phosphorpentachlorid wurden unter Rühren bei Raumtemperatur zu einer Mischung von 41,4 Teilen 6-Methyl-3-ethoxy-6H-1,2,4,6-thiatriazin(5)on-1,1-dioxid in 100 Teilen 1,2-Dichlorethan und 100 Teilen Phosphoroxyhalogenid gegeben. Die Reaktionsmischung wurde 12 Stunden unter Rückfluß gerührt. Nach dem Einengen im Vakuum wurden 43,5 Teile (96,5 % der Theorie) 5-Chlor-6-methyl-3-ethoxy-6H-1,2,4,6-thiatriazin-1,1-dioxid mit Fp. 75-80 °C erhalten.

Vorschrift 6

50 Teile Phosphorpentachlorid wurden zu einer Mischung von 44 Teilen 6-n-Propyl-3-methoxy-6H-1,2,4,6-thiatriazin(5)-on-1,1-dioxid und 268 Teilen Phosphoroxychlorid unter Rühren bei 22 °C gegeben und innerhalb 20 Minuten auf 110 °C erhitzt. Nach 7 Stunden Rühren unter Rückfluß wurde die Reaktionsmischung im Vakuum eingeengt, wobei 45 Teile öliges 5-Chlor-6-n-propyl-3-methoxy-6H-1,2,4,6-thiatriazin-1,1-dioxid erhalten wurden ; NMR : ($CDCl_3$) N-$CH_2$ 4.0-4.28

Durch Destillation bei 125-130 °C/0,01 mbar wurden 40,3 Teile (84 % der Theorie) Reinprodukt erhalten.

Vorschrift 7

Eine Suspension von 25 Teilen 6-Ethyl-3-methylmercapto-6H-1,2,4,6-thiatriazin(5)on-1,1-dioxid in 2,5 Teilen DMF und 245 Teilen 1,2-Dichlorethan wurde während 14 Stunden unter rühren bei 83 °C mit Phosgen begast. Nach dem Einengen im Vakuum wurden 27 Teile viskoses Öl isoliert, das nach dem NMR-Spektrum ca. 45 % 5-Chlor-6-ethyl-3-methylmercapto-6H-1,2,4,6-thiatriazin-1,1-dioxid enthielt. Eine Probe wurde bei 136-144 °C/0,01 mbar destilliert ; NMR : ($CDCl_3$) N-$CH_2$ 4.04-4.42 δ (q), $CH_3S$ 2.52 δ (s).

Die folgenden Beispiele betreffen die Herstellung der neuen Stoffe.

Beispiel 1

6-Methyl-3-n-propoxy-5-p-nitrophenoxy-6H-1,2,4,6-thiatriazin-1,1-dioxid (Nr. 3)

13,9 Teile p-Nitrophenol in 40 Teilen Methylenchlorid und 10,2 Teile Triethylamin wurden gleichzeitig über 2 Zuführungen unter Rühren bei 20 bis 25 °C in eine Lösung von 23,9 Teilen 5-Chlor-6-methyl-3-n-propoxy-6H-1,2,4,6-thiatriazin-1,1-dioxid in 180 Teilen Methylenchlorid innerhalb 10 Minuten eingeführt. Das Reaktionsgemisch wurde 3 Stunden unter Rückfluß gerührt und nach dem Abkühlen mit Wasser gewaschen. Anschließend wurde 3 x mit verd. Natronlauge gewaschen, über Magnesiumsulfat getrocknet und im Vakuum eingeengt. Es wurden 31 Teile 6-Methyl-3-n-propoxy-5-p-nitrophenoxy-6H-1,2,4,6-thiatriazin-1,1-dioxid mit Fp. 129 bis 131 °C erhalten.

Beispiel 2

6-Methyl-3-ethoxy-5-allyloxy-6H-1,2,4,6-thiatriazin-1,1-dioxid (Nr. 166)

7 Teile Allylalkohol und 12,9 Teile 2,6-Dimethylpyridin wurden gleichzeitig über 2 Zuführungen unter Rühren innerhalb 6 Minuten bei 25-35 °C in einer Mischung von 22,6 Teilen 5-Chlor-6-methyl-3-ethoxy-6H-1,2,4,6-thiatriazin-1,1-dioxid in 140 Teilen Tetrahydrofuran eingeführt. Nach 30 Minuten Rühren bei 50 °C wurde das Reaktionsgemisch eingeengt, in Methylenchlorid aufgenommen und 3x mit 10 % Natriumcarbonatlösung gewaschen. Anschließend wurde die Lösung über Magnesiumsulfat getrocknet und im Vakuum eingeengt, wobei 14 Teile 6-Methyl-3-ethoxy-5-allyloxy-6H-1,2,4,6-thiatriazin-1,1-dioxid mit Fp. 56-60 °C erhalten wurden.

In entsprechender Weise wurden die folgenden neuen Verbindungen erhalten.

(Siehe Tabellen Seite 6 ff.)

| Verbindung Nr. | $R^1$ | X | Y | $R^2$ | $R^3$ | $Fp[^oC]/n_D^{25}$ |
|---|---|---|---|---|---|---|
| 5 | $CH_3$ | O | O | $CH_3$ | $CH_3$ | 100-104 |
| 6 | $CH_3$ | O | O | $C_2H_5$ | $CH_3$ | 108-112 |
| 7 | $CH_3$ | O | O | $C_2H_5$ | $C_2H_5$ | 77- 80 |
| 9 | $CH_3$ | O | O | $CH_2-CH=CH_2$ | $C_2H_5$ | 69- 72 |
| 10 | $CH_3$ | S | O | $CH_3$ | $CH_3$ | 129-133 |
| 11 | $CH_3$ | S | S | $CH_3$ | $CH_3$ | |
| 13 | $CH_3$ | O | O | $CH_2-CH=CH_2$ | H | |
| 14 | $C_2H_5$ | O | O | $CH_3$ | $CH_3$ | |
| 15 | $C_2H_5$ | O | S | $C_2H_5$ | $CH_3$ | |
| 16 | $C_2H_5$ | O | O | $C_2H_5$ | $C_2H_5$ | |
| 17 | $C_2H_5$ | O | O | $CH_2-CH=CH_2$ | $C_2H_5$ | |
| 18 | $C_2H_5$ | O | O | 1-Propin-yl-3 | $CH_3$ | 78- 81 |
| 19 | $C_2H_5$ | S | O | $CH_2-CH=CH_2$ | $CH_3$ | |
| 20 | $C_2H_5$ | O | S | $CH_2-CH=CH_2$ | $CH_3$ | |
| 21 | $C_2H_5$ | O | O | $CH=CH-CH_2Cl$ | $CH_3$ | |
| 22 | $C_2H_5$ | O | S | $CH=C(Cl)-CH_3$ | $CH_3$ | |
| 23 | $n-C_3H_7$ | O | O | CH2-CH2-OCH3 | CH3 | |

| Verbindung Nr. | $R^1$ | X | Y | $R^2$ | $R^3$ | Fp[°C]/$n_D^{25}$ |
|---|---|---|---|---|---|---|
| 24 | $n-C_3H_7$ | O | O | 1-Chlor-2-butin-yl-4 | $CH_3$ | |
| 27 | $n-C_3H_7$ | O | O | 1-Chlor-3-butin-yl-2 | $C_2H_5$ | |
| 28 | $CH_3$ | O | S | 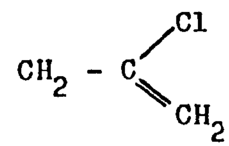 | $CH_3$ | |
| 29 | $C_2H_5$ | O | O | $CH_2-CH=CH-\overset{O}{\overset{\|}{C}}-CH_3$ | $CH_3$ | |
| 30 | $CH_3$ | O | O | $CH_2-\overset{O}{\overset{\|}{C}}-CH_3$ | $CH_3$ | |
| 31 | $CH_3$ | O | O | $CH_2-CH_2-S-CH_3$ | $CH_3$ | |
| 33 | $n-C_4H_9$ | O | O | $C_2H_5$ | $CH_3$ | |
| 36 | $CH_3$ | O | O | $\overset{CH_3}{\overset{\mid}{C}H}-CO_2C_2H_5$ | $CH_3$ | 1.4829 |
| 37 | $CH_3$ | O | O | 4-CF3-phenyl | CH3 | |

| Verbindung Nr. | $R^1$ | X | Y | $R^2$ | $R^3$ | Fp[°C]/$n_D^{25}$ |
|---|---|---|---|---|---|---|
| 39 | $CH_3$ | O | S | 3-Chlorphenyl | $CH_3$ | |
| 40 | $CH_3$ | O | O | n-$C_3H_7$ | $CH_3$ | 50- 54 |
| 41 | $C_2H_5$ | O | O | n-$C_4H_9$ | $C_2H_5$ | |
| 42 | $CH_3$ | O | O | 4-Methylmercaptophenyl | $C_2H_5$ | |
| 43 | $CH_3$ | O | O | 4-Methoxyphenyl | $CH_3$ | |
| 44 | $CH_3$ | O | O | $CH_3$ | $C_2H_5$ | 102-104 |
| 46 | n-$C_4H_9$ | O | O | $C_2H_5$ | $C_2H_5$ | |
| 47 | $CH_3$ | O | S | $CH_3$ | $CH_3$ | 125-128 |
| 48 | $CH_3$ | O | O | 2-Butin-yl-4 | $CH_3$ | |
| 49 | $CH_3$ | O | O | 1-Butin-yl-3 | $CH_3$ | |
| 50 | $C_2H_5$ | S | O | 1-Butin-yl-3 | $CH_3$ | |
| 51 | $CH_3$ | O | O | $CH_2$-$CH$=$CH_2$ | $CH_3$ | 55- 57 |

| Verbindung Nr. | $R^1$ | X | Y | $R^2$ | $R^3$ | Fp[°C]/$n_D^{25}$ |
|---|---|---|---|---|---|---|
| 59 | $C_2H_5$ | O | S | n-$C_4H_9$ | $C_2H_5$ | |
| 60 | n-$C_3H_7$- | O | O | $C_2H_5$ | $CH_3$ | 49- 51 |
| 61 | $CH_3$ | O | O | 4-Methylphenyl | $C_2H_5$ | |
| 62 | $CH_3$ | O | O | 4-$CF_3S$-phenyl | $CH_3$ | |
| 63 | $C_2H_5$ | S | O | $CH_3$ | $CH_3$ | |
| 64 | $C_2H_5$ | O | O | $C_2H_5$ | 1-$C_3H_7$ | 40- 46 |
| 66 | $C_2H_5$ | O | O | $CH_3$ | 1-$C_3H_7$ | 74- 78 |
| 69 | $C_2H_5$ | O | O | $C_2H_5$ | $CH_3$ | 72- 75 |
| 74 | $CH_3$ | S | O | $CH_2$-CH=$CH_2$ | $CH_3$ | 90 - 93 |
| 75 | $CH_3$ | O | S | 1-Propin-yl-3 | $CH_3$ | |

| Verbindung Nr. | $R^1$ | X | Y | $R^2$ | $R^3$ | $Fp[^{o}C]/n_D^{25}$ |
|---|---|---|---|---|---|---|
| 79 | $CH_3$ | O | O | 1-Propin-yl-3 | H | |
| 80 | $C_2H_5$ | O | O | $CH_2-CH=CH\overset{O}{\overset{\|}{C}}-CH_3$ | $C_2H_5$ | |
| 84 | $CH_3$ | O | O | $CH_3$ | Cyclohexyl | |
| 89 | $CH_3$ | O | O | 4-Nitrophenyl | $CH_3$ | 235-240 |
| 90 | $CH_3$ | O | O | 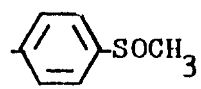 | $CH_3$ | |
| 91 | $C_2H_5$ | S | O | 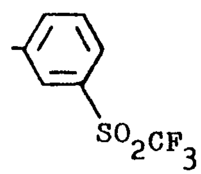 | $CH_3$ | |
| 92 | $C_2H_5$ | O | O | 4-Cyanophenyl | $CH_3$ | |

| Verbindung Nr. | $R^1$ | X | Y | $R^2$ | $R^3$ | Fp[°C]/$n_D^{25}$ |
|---|---|---|---|---|---|---|
| 96 | $CH_3$ | O | O | 2-Methyl-4,6-dinitro-phenyl | $CH_3$ | 160-163 |
| 97 | $C_2H_5$ | O | S | 4-Nitrophenyl | $CH_3$ | |
| 98 | $n-C_3H_7$ | O | O | 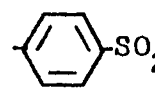 $-SO_2CH_3$ | $CH_3$ | |
| 99 | $CH_3$ | O | O | 2,4-Dichlorphenyl | $CH_3$ | 189-194 |
| 100 | $CH_3$ | O | O | 3,5-Dichlorphenyl | $C_2H_5$ | |
| 101 | $CH_3$ | O | O | 2,3-Dichlorphenyl | $n-C_3H_7$ | |
| 103 | $CH_3$ | O | O | 2,4.6-Trichlorphenyl | $CH_3$ | |
| 105 | $C_2H_5$ | O | O | 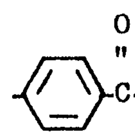 $-C-NHCH_3$ | $C_2H_5$ | |

| Verbindung Nr | $R^1$ | X | Y | $R^2$ | $R^3$ | Fp[°C]/$n_D^{25}$ |
|---|---|---|---|---|---|---|
| 107 | $CH_3$ | O | O | 2,4-Dinitro-6-tertiär-butylphenyl | $CH_3$ | 156-161 |
| 110 | $CH_3$ | O | O | 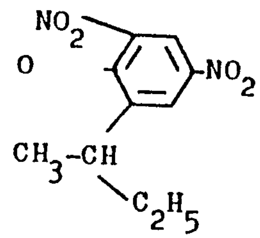 | $CH_3$ | 151-156 |
| 113 | $CH_3$ | O | O | 2,6-Dibrom-4-cyano-phenyl | $CH_3$ | 242-246 |

| Verbindung Nr. | $R^1$ | X | Y | $R^2$ | $R^3$ | Fp[ °C]/$n_D^{25}$ |
|---|---|---|---|---|---|---|
| 116 | $C_2H_5$ | S | O | Benzyl | $CH_3$ | |
| 117 | $CH_3$ | O | O | 2,6-Dijod-4-cyanophenyl | $CH_3$ | 276-281 |
| 118 | $CH_3$ | O | O | 2-Nitrophenyl | $C_2H_5$ | |
| 119 | $CH_3$ | O | O | 3-Nitrophenyl | $CH_3$ | 213-218 |
| 121 | $CH_3$ | S | O | 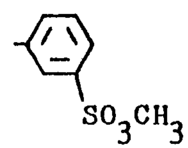 | $CH_3$ | |
| 123 | $CH_3$ | O | O | 4-Chlorphenyl | $CH_3$ | 199-202 |

| Verbindung Nr. | $R^1$ | X | Y | $R^2$ | $R^3$ | Fp[°C]/$n_D^{25}$ |
|---|---|---|---|---|---|---|
| 126 | $CH_3$ | O | O | 4-Nitrophenyl | $C_2H_5$ | 176-178 |
| 129 | $CH_3$ | O | O | 4-tert.-butylphenyl | $CH_3$ | 155-158 |
| 130 | $C_2H_5$ | O | S | 2,6-Dimethylphenyl | $CH_3$ | |
| 131 | $CH_3$ | O | O | 4-Chlorbenzyl | $CH_3$ | |
| 132 | $CH_3$ | O | O | 4-Cyanophenyl | $CH_3$ | 219-221 |
| 133 | $CH_3$ | O | O | 3,4-Difluorphenyl | $CH_3$ | |
| 134 | $CH_3$ | O | O | 3-tert.-butylphenyl | $CH_3$ | 113-116 |
| 135 | $C_2H_5$ | O | O | 4-tert.-butylphenyl | $CH_3$ | |
| 136 | $CH_3$ | O | O | 2,4-Dichlor-6-methyl-phenyl | $CH_3$ | 194-196 |

| Verbindung Nr. | $R^1$ | X | Y | $R^2$ | $R^3$ | $Fp[^oC]/n_D^{25}$ |
|---|---|---|---|---|---|---|
| 137 | $C_2H_5$ | O | O | 2-Chlor-4-nitrophenyl | $CH_3$ | |
| 138 | $CH_3$ | O | O | 4-Nitrophenyl | H | |
| 139 | $CH_3$ | O | O | 4-$CF_3$-2-chlorphenyl | $CH_3$ | 178-179 |
| 140 | $CH_3$ | O | O | 3-Chlor-4-methoxyphenyl | H | |
| 141 | $C_2H_5$ | O | O | 3-$CF_3$-phenyl | H | |
| 142 | $CH_3$ | O | O | 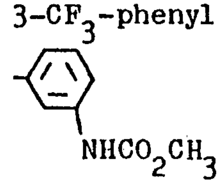 | $CH_3$ | 199-204 |
| 143 | $CH_3$ | O | O | 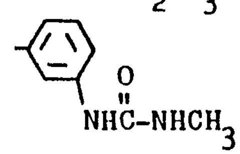 | $CH_3$ | 216-220 |
| 145 | $CH_3$ | S | O | 3,4-Dichlorbenzyl | $CH_3$ | |
| 146 | $C_2H_5$ | O | O | 4-Nitrophenyl | $CH_3$ | 146-150 |

| Verbindung Nr. | $R^1$ | X | Y | $R^2$ | $R^3$ | Fp[°C]/$n_D^{25}$ |
|---|---|---|---|---|---|---|
| 151 | $C_2H_5$ | O | S | Benzyl | $CH_3$ | |
| 153 | $CH_3$ | O | O | 4-Nitrophenyl | n-$C_3H_7$ | 152-155 |
| 154 | $CH_3$ | S | O | Benzyl | $CH_3$ | |
| 155 | $CH_3$ | O | O | Benzyl | $CH_3$ | 141-145 |
| 156 | $CH_3$ | O | O | 3-$CF_3O$-Phenyl | $CH_3$ | |
| 157 | $CH_3$ | O | O | sek.-Butyl | $CH_3$ | 1.4904 |
| 158 | $CH_3$ | O | O | 1-Propin-yl-3 | $CH_3$ | 98-102 |
| 159 | $CH_3$ | O | O | 1-Propin-yl-3 | $C_2H_5$ | |
| 160 | $C_2H_5$ | O | O | 1-Propin-yl-3 | $C_2H_5$ | |
| 161 | $C_2H_5$ | O | O | Benzyl | $CH_3$ | 73- 75 |
| 162 | $CH_3$ | O | O | 2,6-Dichlorbenzyl | $CH_3$ | 104-107 |
| 163 | n-$C_3H_7$ | O | O | $CH_2$-CH=$CH_2$ | $CH_3$ | 43- 47 |
| 164 | n-$C_3H_7$ | O | O | 1-Propin-yl-3 | $CH_3$ | 98-103 |
| 165 | $C_2H_5$ | O | O | 2,6-Dichlorbenzyl | $CH_3$ | 119-123 |
| 166 | $C_2H_5$ | O | O | Allyl | $CH_3$ | 56- 60 |

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser ; Trägerstoffe wie natürliche Gesteinsmehle (z. B. Kaoline, Tonerden, Talkum, Kreide) und synthetische Gesteinsmehle (z. B. hochdisperse Kieselsäure, Silikate) ; Emulgiermittel wie nichtionogene und anionische Emulgatoren (z. B. Polyoxyethylen — Fettalkohol — Ether, Alkylsulfonate und Arylsulfonate) und Dispergiermittel, wie Lignin, Sulfitablaugen und Methylcellulose.

Zur Aktivierung der herbiziden Wirkung können Netz- und Haftmittel sowie nicht-phytotoxische Öle und Ölkonzentrate zugesetzt werden.

Die herbiziden Mittel enthalten im allgemeinen zwischen 0,1 und 95 Gew.-% Wirkstoff, vorzugsweise zwischen 0,5 und 90.

Die Aufwandmengen liegen je nach Zusammensetzung und Wachstumsstadien der Unkrautflora zwischen 0,1 und 15, vorzugsweise jedoch zwischen 0,2 und 5 kg Wirkstoff pro Hektar, wobei für eine totale Pflanzenvernichtung die höheren Aufwandmengen zu verwenden sind.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind :

I. Man vermischt 90 Gewichtsteile der Verbindung des Beispiels 1 mit 10 Gewichtsteilen N-methyl-α-pyrrolidon und erhält eine Lösung, die zur Anwendung in Form kleinster Tropfen geeignet ist.

II. Man vermischt 80 Gewichtsteile der Verbindung des Beispiels 2 mit 20 Gewichtsteilen Cyclohexanon. Die Mischung kann in Form feiner Tropfen versprüht werden.

III. 20 Gewichtsteile der Verbindung des Beispiels 1 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IV. 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.-% des Wirkstoffs enthält.

V. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VI. 20 Teile der Verbindung des Beispiels 1 werden mit 2 Teilen Calciumsalz der Dodecylbenzolsulfonsäure, 8 Teilen Fettalkoholpolyglykolether, 2 Teilen Natriumsalz eines Phenolsulfonsäureharnstoff-formaldehyd-Kondensates und 68 Teilen eines paraffinischen Mineralöls innig vermischt. Man erhält eine stabile ölige Dispersion.

Die neuen Verbindungen können allein, unter sich oder mit anderen Herbiziden sowie auch noch mit weiteren Pflanzenschutzmitteln gemischt und gemeinsam ausgebracht werden, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden.

Der Einfluß von Vertretern der neuen und herbiziden substituierten 6H-1,2,4,6-thiatriazin-1,1-dioxide auf das Wachstum von erwünschten und unerwünschten Pflanzen wird anhand von Gewächshausversuchen aufgezeigt.

Als Kulturgefäße dienten Plastikblumentöpfe mit 300 $cm^3$ Inhalt und lehmiger Sand mit etwa 1,5 % Humus als Substrat. Bei Cyperus esculentus, Glycine max und bei Oryza sativa wurde etwas Torf (peat) zugesetzt, um ein besseres Wachstum zu gewährleisten. Die Samen der in der nachfolgenden Tabelle 1 aufgeführten Pflanzen säte man nach Arten getrennt flach ein. Bei Cyperus esculentus nahm man angekeimte Knollen (tubers). Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt.

Dabei variierten die Aufwandmengen je nach Wirkstoff. Es wurden Dosierungen von 0,5, 1,0, 2,0 und 4,0 kg/ha Aktivsubstanz eingesetzt. Nach dem Aufbringen beregnet man die Gefäße leicht, um Keimung und Wachstum in Gang zu bringen und die chemischen Mittel zu aktivieren. Danach deckte man die Gefäße mit durchsichtigen Plastikhauben ab, bis die Pflanzen angewachsen waren. Dieses Abdecken

bewirkte ein gleichmäßiges Keimen der Testpflanzen, sofern dies nicht durch die Chemikalien beeinträchtigt wurde.

Zum Zwecke der Nachauflaufbehandlung zog man die Testpflanzen je nach Wuchsform erst bis zu einer Wuchshöhe von 3 bis 10 cm an und behandelte sie dann. Zur Nachauflaufbehandlung wurden entweder direkt gesäte und in den gleichen Gefäßen aufgewachsene Pflanzen ausgewählt, oder sie wurden erst als Keimpflanzen separat angezogen und einige Tage vor der Behandlung in die Versuchsgefäße transportiert. Die Aufwandmengen für die Nachauflaufbehandlungen waren ebenfalls bei einzelnen Wirkstoffen verschieden. Sie beliefen sich auf 0,5 und 1,0 oder 2,0 kg/ha Aktivsubstanz.

Als Vergleichsmittel in diesen Versuchen diente die Verbindung A

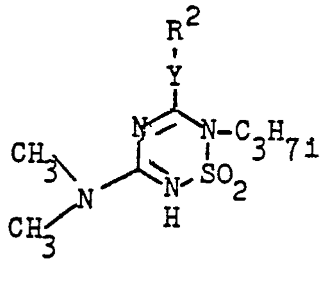

(bekannt aus DE-OS 25 08 832).

Bei der Nachauflaufbehandlung unterblieb eine Abdeckung. Die Aufstellung aller Versuche erfolgte im Gewächshaus, wobei für wärmeliebende Arten wärmere Bereiche (20 bis 35 °C) und für solche gemäßigter Klimate 10 bis 20 °C bevorzugt wurden. Die Vergleichsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Aufgang und 100 kein Aufgang der Pflanzen bzw. völlige Zerstörung zumindest der oberirdischen Sproßteile.

Die Ergebnisse zeigten, daß die neuen erfindungsgemäßen Verbindungen geeignet sind zur Bekämpfung von breitblättrigen unerwünschten Pflanzen sowie von annuellen und perennierenden Cyperaceen in Kulturpflanzen oder auf Totalflächen bei Vor- und Nachauflaufbehandlung. Auch eine Wirkung gegen unerwünschte Gräser ist erwähnenswert.

(Siehe Tabelle 1 Seite 19 f.)

Tabelle 1 : Liste der Pflanzennamen

| Botanischer Name | Abkürzung in Tabelle | Deutscher Name | Englischer Name |
|---|---|---|---|
| Chenopodium album | Chenop. alb. | weißer Gänsefuß | lambsquarters |
| Cyperus spp. (C.iria, Cerax, C. difformis) | – | Riedgräser | annual sedges |
| Cyperus esculentus | Cyperus escul. | Erdmandel | yellow untsedge |
| Datura stramonium | Datura stram. | Stechapfel | jimson weed |
| Glycine max | – | Sojabohnen | soybeans |
| Gossypium hirsutum | – | Baumwolle | cotton |
| Matricaria spp. | Matric. spp. | Kamillearten | chamomile spp. |
| Nicandra physaloides | Ncandra physal. | | apple of peru |
| Oryza sativa | – | Reis | rice |
| Solanum nigrum | Solan. nigr. | schwarzer Nachtschatten | black nightshade |
| Zea mays | – | Mais | indian corn, maize |

Bei einem Versuch zur Prüfung der herbiziden Wirkung bei Nachauflaufbehandlung im Gewächshaus zeigten die neuen Verbindungen 51, 146 und 69 bei Aufwandmengen von 0,5 und 1,0 kg Wirkstoff je ha eine bessere selektive herbizide Wirkung als der bekannte Wirkstoff A.

Bei einem Versuch zur Bekämpfung unerwünschter breitblättriger Pflanzen bei Sojabohnen bei Vorauflaufanwendung im Gewächshaus zeigten die neuen Verbindungen 113, 36, 142, 119, 129, 136, 139 und 123 bei Aufwandmengen von 0,5 und 1,0 kg Wirkstoff je ha eine bessere selektive herbizide Wirkung als der bekannte Wirkstoff A.

Bei einem Versuch zur Bekämpfung von Cyperaceen im Reis bei Vorauflaufanwendung im Gewächshaus zeigten die neuen Wirkstoffe 40, 132, 69, 89 und 155 bei Aufwandmengen von 4,0 und 2,0 kg Wirkstoff je ha eine gute selektive herbizide Wirkung.

Bei einem Versuch zur selektiven Bekämpfung von breitblättrigen unerwünschten Pflanzen in Kulturen bei Vorauflaufanwendung im Gewächshaus zeigten die neuen Wirkstoffe 143, 139 und 110 bei Aufwandmengen von 1,0 und 2,0 kg Wirkstoff je ha eine gute selektive herbizide Wirkung.

Auch bei selektiver Bekämpfung von Cyperus esculentus in Reis bei Nachauflaufanwendung im Gewächshaus zeigten z. B. die Verbindungen 89 und 155 eine ausgezeichnete Wirkung.

Sind gewisse Kulturpflanzen gegenüber den Wirkstoffen weniger tolerant, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post directed, lay-by). In Anbetracht der guten Verträglichkeit und der Vielseitigkeit der Applikationsmethoden können die erfindungsgemäßen Herbizide oder diese enthaltende Mittel noch in einer weiteren großen Zahl von Kulturpflanzen zur Beseitigung unerwünschten Pflanzenwuchses eingesetzt werden. Die Aufwandmengen können dabei zwischen 0,1 und 15 kg/ha und mehr schwanken.

In Betracht kommen beispielsweise folgende Kulturen :

(Siehe Tabellen Seite 21 ff.)

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. naprobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor-Färbedistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea cane phora, Coffea liberica) | Kaffee | coffee plants |

| Botanischer Name | Deutscher Name | Englischer Name |
|---|---|---|
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactuca sativa | Kopfsalat | lettuce |
| Lens culinaris | Linse | lentils |
| Linum usitatissimum | Faserlein | flax |
| Lycopersiocon lycopersicum | Tomate | tomato |
| Malus spp. | Apfel | apple trees |

| Botanischer Name | Deutscher Name | Englischer Name |
|---|---|---|
| Manihot esculenta | Maniok | cassava |
| Medicago sativa | Luzerne | alfalfa (lucerne) |
| Mentha piperita | Pfefferminze | peppermint |
| Musa spp. | Obst- u. Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beens, dry beans |
| Pennisetum glaucum | Perl- oder Rohrkolbenhirse | |
| Petroselinim crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |

| Botanischer Name | Deutscher Name | Englischer Name |
|---|---|---|
| Prunus dulcis | Mandelbaum | almond trees |
| Prunus persica | Pfirsich | peach trees |
| Prunus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potato |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium carymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Zur Verbreiterung des Wirkungsspektrums und zur Erzielung auch synergistischer Effekte können die neuen Wirsktoffe sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazin-derivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt :

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-(3-trifluormethylphenyl)-3(2H)-pyridazinon
5-Methylamino-4-chlor-2-(3-α,α,β,β-tetrafluoräthoxyphenyl-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3-trifluormethylphenyl)-3(2H)-pyridazinon
5-Methoxy-4-chlor-2-(3-trifluormethylphenyl)-3(2H)-pyridazinon
5-Amino-4-brom-2-(3-methylphenyl)-3(2H)-pyridazinon

3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl)-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Cyan-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-methylethyl)-1H-(pyridino-[3,2-e]-2,1,3-thiadiazin-(4)-on-2,2-dioxid

N-(1-Ethylpropyl)-2,6-dinitro-3,4-dimethylanilin
N-(1-Methylethyl)-N-ethyl-2,6-dinitro-4-trifluormethylanilin
N-n. Propyl-N-β-chlorethyl-2,6-dinitro-4-trifluormethylanilin
N-n. Propyl-N-cyclopropylmethyl-2,6-dinitro-4-trifluormethyl-anilin
N-Bis-(n. Propyl)-2,6-dinitro-3-amino-4-trifluormethylanilin
N-Bis-(n. propyl)-2,6-dinitro-4-methyl-anilin
N-Bis-(n. propyl)-2,6-dinitro-4-methylsulfonyl-anilin
N-Bis-(n. propyl)-2,6-dinitro-4-aminosulfonyl-anilin
Bis-(β-chlorethyl)-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert. butyl-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionanilid

Ethyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl-carbamat

Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-(N'-3-chlor-4-fluorphenylcarbamoyloxy)-phenyl)-carbamat
Ethyl-N-(3-(N'-3,4-difluorphenylcarbamoyloxy)-phenyl)-carbamat
Methyl-N-(3-(N'-3,4-difluorphenylcarbamoyloxy)-phenyl)-carbamat

N-3-(4-Fluorphenoxycarbonylamino)-phenylcarbaminsäure-methylester
N-3-(2-Methylphenoxycarbonylamino)-phenylcarbaminsäure-ethylester
N-3-(4-Fluorphenoxycarbonylamino)-phenylthiolcarbaminsäuremethylester
N-3-(2,4,5-Trimethylphenoxycarbonylamino)-phenylthiolcarbaminsäure-methylester
N-3-(Phenoxycarbonylamino)-phenylthiolcarbaminsäure-methylester

N,N-Diethyl-thiolcarbaminsäure-p-chlorbenzylester
N,N-Di n. propyl-thiolcarbaminsäure-ethylester
N,N-Di n. propyl-thiolcarbaminsäure-n. propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec. butyl-thiolcarbaminsäure-ethylester
N,N-Di-sec. butyl-thiolcarbaminsäure-benzylester
N-Ethyl-N-cyclohexyl-thiolcarbaminsäure-ethylester
N-Ethyl-N-bicyclo-[2,2,1]-heptyl-thiolcarbaminsäureäthylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-3methylhexahydro-1-H-azepin-1-carbothiolat
S-Benzyl-2,3-dimethylhexahydro-1-H-azepin-1-carbothiolat
S-Ethyl-3methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz
α-Methyl,α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure
2,3,5-Triiodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephtalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureäthylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäuremethylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäuremethylester
2-(N-Benzoyl-3-chlor-4-fluorphenylamino)-propionsäure-isopropylester
2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin

2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert. butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert. butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert. butyl-3-methylthio-4,5-dihydro-1,2,4-triazin-5-on
1-Methyl-3-cyclohexyl-6-dimethylamino-1,3,5-triazin-2,4-dion

3-tert. Butyl-5-chlor-6-methyluracil
3-tert. Butyl-5-brom-6-methyluracil
3-Isopropyl-5-brom-6-methyluracil
3-sec. Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')-]-ethan (Salze)
1-(4-Chlorphenoxy-3,3-dimethyl-1(1H-1,2,4-triazol-1-yl)-2-butanon
N,N-Diallylchloracetamid
N-Isopropyl-2-chloracetanilid
N-(Butin-1-yl-3)-2-chloracetanilid

2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid
2,6-Dimethyl-N-(pyrazon-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxalan-2-yl-methyl)-2-chloracetanilid
2,6-Dimethyl-N-(2-methoxymethyl)-2-chloracetanilid
2,6-Dimethyl-N-(isobutoxymethyl)-2-chloracetanilid
2,6-Diethyl-N-(methoxymethyl)-2-chloracetanilid
2,6-Diethyl-N-(n. butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(2-n-propoxyethyl)-2-chloracetanilid

2-(2-Methyl-4-chlorphenoxy-N-methoxy-acetamid
2-(α-Naphtoxy)-N,N-diethylpropionamid
2,2-Diphenyl-N,N-dimethylacetamid
α(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionnsäure-3,4-dichloranilid
Cyclopropancarbonsäure-3,4-dichloranilid
Methacrylsäure-3,4-dichloranilid
2-Methylpentancarbonsäure-3,4-dichloranilid

5-Acetamido-2,4-dimethyltrifluormethan-sulfonanilid
5-Acetamido-4-methyl-trifluormethan-sulfonanilid
2-Propionyl-amino-4-methyl-5-chlor-thiazol
O-(Methylsulfonyl)-glykolsäure-N-ethoxymethyl-2,6-dimethyl-anilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
3,5-Dibrom-4-hydroxy-O-2-Cyan-4-nitrophenylbenzaldoxim (Salze)
Pentachlorphenol-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-tert. Butylcarbamoyl-oxyphenyl)-4-methyl-1,2,4-oxadiazolidin-3,5-dion
2-(3-iso-Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-ethoxy-2,3-dihydro-3,3-dimethyl-5-benzofuranyl-methansulfonat
2-ethoxy-2,3-dihydro-3,3-dimethyl-5-benzofuranyl-dimethyl-aminosulfonat
2-ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)
2-sec. Butyl-4,6-dinitrophenol (Salze)
2-sec. Butyl-4,6-dinitrophenol-acetat
2-tert. Butyl-4,6-dinitrophenol-acetat
2-tert. Butyl-4,6-dinitrophenol (Salze)
2-tert. Butyl-5-methyl-4,6-dinitrophenol (Salze)
2-tert. Butyl-5-methyl-4,6-dinitrophenol-acetat

2-sec. Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-chlorphenyl)-1-benzoyl-3,3-dimethyl-harnstoff
1-(4-chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n. butyl-harnstoff
1-(4-i-Propylphenyl)-3,3-dimethyl-harnstoff
1-(3-Trifluormethylphenyl)-3,3-dimethyl-harnstoff
1-(α,α,β,β-Tetrafluoräthoxyphenyl)-3,3-dimethyl-harnstoff

1-(3-tert. Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff

1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert. Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-iso-butylamid
1,2-Dimethyl-3,5-diphenylpyrazolium-methylsulfat
1,2,4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenylsulfonyl)-oxyl]-pyrazol
2,3,5-Trichlor-pyridinol-(4)
1-Methyl-3-phenyl-5-(3′-trifluormethylphenyl)-pyridon-(4)
1-Methyl-4-phenyl-pyridiniumchlorid
1,1-Dimethylpyridiniumchlorid
1,1′-Dimethyl-4,4′-dipyridylium-di(methylsulfat)3-Phenyl-4-hydroxy-6-chlorpyridazin
1,1′-Di(3,5-dimethylmorpholin-carbonylmethyl)-4,4′-dipyridylium-dichlorid
1,1′-Ethylen-2,2′-dipyridylium-dibromid
3-[1(N-Ethoxyamino)-propyliden]-6-ethyl-3,4-dihydro-2-H-pyran-2,4-dion
3-[1-(N-Allyloxyamino)-propyliden]-6-äthyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide).

α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
0,0-Diisopropyl-5-(2-benzosulfonylamino-ethyl)-phosphordithionat
2,3-Dihydro-5,6-dimethyl-1,4-dithiin-1,1,4,4-tetraoxid
5-tert. Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon Natriumchlorat

Ammoniumrhodanid
Calciumcyanamid
2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert. Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Iodpyridyl-2-oxy)-phenoxy]-propionsäureethylester
2-[4-(5'-Iodpyridyl-2-oxy)-phenoxy]-propionsäure-n-butylester
2-Chlor-4-trifluormethylphenyl-3'-(2-fluorethoxy)-4'-nitrophenylether
2-Chlor-4-trifluormethylphenyl-3(ethoxycarbonyl) methylthio-4-nitropenylether
2,4,6-Trichlorphenyl-3'-ethoxycarbonyl)-methylthio-4-nitrophenylether
2-[1-(N-ethoxyamino)-butyliden]-5-(2-ethylthiopropyl)-3-hydroxy-cyclohexen-(2)-on-(1) (Salze)
2-[1-(N-ethoxyamino)-butyliden]-5-(2-phenylthiopropyl)-3-hydroxy-cyclohexen-(2)-on-(1) (Salze)

4-[4-(4'-Trifluormethyl)-phenoxy]-penten-2-carbonsäureethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-α,α,β-trifluor-β-bromethoxyphenyl)-3-(2H)-pyridazinon
2,4-Dichlorphenyl-3'-ethoxy-ethoxy-ethoxy-4'-nitrophenylether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1(3-Chlor-4-ethoxyphenyl)-3,3-dimethylharnstoff
2-Methyl-4-Chlorphenoxy-thioessigsäureethylester

**Ansprüche** (für die Vertragsstaaten : BE, CH, DE, FR, GB, IT, LI, NL, SE)

1. 6H-1,2,4,6-Thiatriazin-1,1-dioxid der Formel

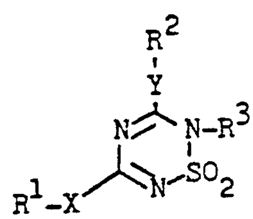

(I)

in der

$R^1$ Methyl, Ethyl, Propyl, Butyl,

$R^2$ Methyl, Ethyl, Propyl, Butyl, Pentyl, Hexyl, Allyl, Methallyl, Propargyl, Butin-1- oder -2-yl, Chlorethyl, Chlorpropyl, Chlorpropargyl, 4-Chlorbutin-1-yl-3, Chlorallyl, Methoxyethyl, Ethoxyethyl, 3-Methoxypropyl, 3-Methoxybutyl, 1-Methoxy-butyl-2, Methylmercaptoethyl, Ethylmercaptoethyl, 3-Methylmercaptopropyl, Methylmercaptobutyl, gegebenenfalls durch Halogen, Methyl, Halogenmethyl, Halogenmethoxy substituierter Benzylrest, Rest des Propionsäureethylesters oder Propanonyl, wenn $R^1$ und $R^3$ = Methyl, Pentenonyl, wenn $R^1$ = Ethyl und $R^3$ = Methyl, oder gegebenenfalls durch Halogen, Methyl, Ethyl, Propyl, Butyl, Halogenmethyl, Cyan, Nitro, Methoxy, Halogenmethoxy, Methoxycarbamoyl, Methylureido, Methylmercapto, Halogenmethylmercapto, Methylsulfinyl, Methylsulfonyl, Halogenmethylsulfinyl oder Halogenmethylsulfonyl substituiertes Phenyl,

$R^3$ Methyl, Ethyl, Propyl und

X und Y Sauerstoff oder Schwefel

bedeuten.

2. Verbindung gemäß Anspruch 1, dadurch gekennzeichnet, daß $R^1$ Methyl, $R^2$ Halogenbenzyl, $R^3$ Methyl, X und Y Sauerstoff bedeuten.

3. Herbizid, enthaltend ein 6H-1,2,4,6-Thiatriazin-1,1-dioxid gemäß Anspruch 1.

4. Herbizid, enthaltend eine Verbindung gemäß Anspruch 2.

5. Herbizid, enthaltend einen festen oder flüssigen Trägerstoff und ein 6H-1,2,4,6-Thiatriazin-1,1-dioxid gemäß Anspruch 1.

6. Verfahren zur Herstellung eines Herbizids, dadurch gekennzeichnet, daß man einen festen oder flüssigen Trägerstoff vermischt mit einem 6H-1,2,4,6-Thiatriazin-1,1-dioxid gemäß Anspruch 1.

7. Verfahren zur Bekämpfung von unerwünschten Plfanzen, dadurch gekennzeichnet, daß man die unerwünschten Pflanzen oder den Boden behandelt mit einer herbizid wirksamen Menge eines 6H-1,2,4,6-Thiatriazin-1,1-dioxids gemäß Anspruch 1.

8. Verfahren zur Herstellung eines 6H-1,2,4,6-Thiatriazin-1,1-dioxids gemäß Anspruch 1, dadurch gekennzeichnet, daß man ein substituiertes 6H-1,2,4,6-Thiatriazin-1,1-dioxid der Formel

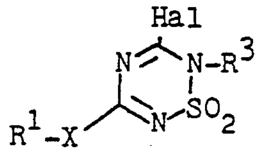

in der $R^1$, $R^3$ und X die im Anspruch 1 genannte Bedeutung haben und Hal für ein Halogenatom steht, mit einer Verbindung der Formel

$$H—Y—R^2$$

in der $R^2$ und Y die im Anspruch 1 genannte Bedeutung haben, oder ihrem Alkali-, Erdalkali- oder Ammoniumsalz, gegebenenfalls in einem inerten organischen Lösungsmittel und gegebenenfalls in Anwesenheit eines Säureakzeptors umsetzt.

**Ansprüche** (für den Vertragsstaat AT)

1. Herbizid, enthaltend ein 6H-1,2,4,6-Thiatriazin-1,1-dioxid der Formel

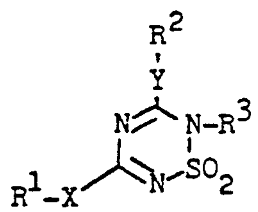

in der

$R^1$ Methyl, Ethyl, Propyl, Butyl

$R^2$ Methyl, Ethyl, Propyl, Butyl, Pentyl, Hexyl, Allyl, Methallyl, Propargyl, Butin-1- oder -2-yl, Chlorethyl, Chlorpropyl, Chlorpropargyl, 4-Chlorbutin-1-yl-3, Chlorallyl, Methoxyethyl, Ethoxyethyl, 3-Methoxypropyl, 3-Methoxybutyl, 1-Methoxy-butyl-2, Methylmercaptoethyl, Ethylmercaptoethyl, 3-Methylmercaptopropyl, Methylmercaptobutyl, gegebenenfalls durch Halogen, Methyl, Halogenmethyl, substituierter Benzylrest, Rest des Propionsäureethylesters oder Propanonyl, wenn $R^1$ und $R^3$ = Methyl, Pentenonyl, wenn $R^1$ = Ethyl und $R^3$ = Methyl, oder gegebenenfalls durch Halogen, Methyl, Ethyl, Propyl, Butyl, Halogenmethyl, Cyan, Nitro, Methoxy, Halogenmethoxy, Methoxycarbamoyl, Methylureido, Methylmercapto, Halogenmethylmercapto, Methylsulfinyl, Methylsulfonyl, Halogenmethylsulfinyl oder Halogenmethylsulfonyl substituiertes Phenyl,

$R^3$ Methyl, Ethyl, Propyl und

X und Y Sauerstoff oder Schwefel

bedeuten.

2. Herbizid gemäß Anspruch 1, dadurch gekennzeichnet, daß $R^1$ Methyl, $R^2$ Halogenbenzyl, $R^3$ Methyl, X und Y Sauerstoff bedeuten.

3. Herbizid, enthaltend einen festen oder flüssigen Trägerstoff und ein 6H-1,2,4,6-Thiatriazin-1,1-dioxid definiert wie in Anspruch 1.

4. Verfahren zur Herstellung eines Herbizids, dadurch gekennzeichnet, daß man einen festen oder flüssigen Trägerstoff vermischt mit einem 6H-1,2,4,6-Thiatriazin-1,1-dioxid definiert wie in Anspruch 1.

5. Verfahren zur Bekämpfung von unerwünschten Pflanzen, dadurch gekennzeichnet, daß man die unerwünschten Pflanzen oder den Boden behandelt mit einer herbizid wirksamen Menge eines 6H-1,2,4,6-Thiatriazin-1,1-dioxids definiert wie in Anspruch 1.

6. Verfahren zur Herstellung eines 6H-1,2,4,6-Thiatriazin-1,1-dioxids definiert wie in Anspruch 1, dadurch gekennzeichnet, daß man ein substituiertes 6H-1,2,4,6-Thiatriazin-1,1-dioxid der Formel

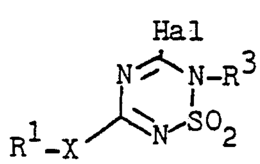

in der $R^1$, $R^3$ und X die im Anspruch 1 genannte Bedeutung haben und Hal für ein Halogenatom steht, mit einer Verbindung der Formel

$$H—Y—R^2$$

in der $R^2$ und Y die im Anspruch 1 genannte Bedeutung haben, oder ihrem Alkali-, Erdalkali- oder Ammoniumsalz, gegebenenfalls in einem inerten organischen Lösungsmittel und gegebenenfalls in Anwesenheit eines Säureakzeptors umsetzt.

**Claims** (for the Contracting States : BE, CH, DE, FR, GB, IT, LI, NL, SE)

1. A 6H-1,2,4,6-thiatriazine-1,1-dioxide of the formula

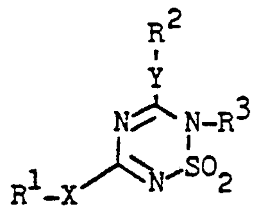

where
$R^1$ is methyl, ethyl, propyl or butyl.
$R^2$ is methyl, ethyl, propyl, butyl, pentyl, hexyl, allyl, methallyl, propargyl, butyn-1-yl, butyn-2-yl, chloroethyl, chloropropyl, chloropropargyl, 4-chlorobut-1-yn-3-yl, chloroallyl, methoxyethyl, ethoxyethyl, 3-methoxypropyl, 3-methoxybutyl, 1-methoxy-but-2-yl, methylmercaptoethyl, ethylmercaptoethyl, 3-methylmercaptopropyl, methylmercaptobutyl, benzyl optionally substituted by halogen, methyl, halomethyl or halomethoxy, or a radical of propionic acid ethyl ester or propanonyl when $R^1$ and $R^3$ are each methyl, or pentenonyl when $R^1$ is ethyl and $R^3$ is methyl, or phenyl optionally substituted by halogen, methyl, ethyl, propyl, butyl, halomethyl, cyano, nitro, methoxy, halomethoxy, methoxycarbamyl, methylureido, methylmercapto, halomethylmercapto, methylsulfinyl, methylsulfonyl, halomethylsulfinyl or halomethylsulfonyl,
$R^3$ is methyl, ethyl or propyl, and
X and Y are oxygen or sulfur.

2. A compound as claimed in claim 1, wherein $R^1$ is methyl, $R^2$ is halobenzyl, $R^3$ is methyl, and X and Y are oxygen.

3. A herbicide containing a 6H-1,2,4,6-thiatriazine-1,1-dioxide as claimed in claim 1.

4. A herbicide containing a compound as claimed in claim 2.

5. A herbicide containing a solid or liquid carrier and a 6H-1,2,4,6-thiatriazine-1,1-dioxide as claimed in claim 1.

6. A process for the production of a herbicide, wherein a solid or liquid carrier is mixed with a 6H-1,2,4,6-thiatriazine-1,1-dioxide as claimed in claim 1.

7. A process for controlling unwanted plants, wherein the unwanted plants or the soil are treated with a herbicidally effective amount of a 6H-1,2,4,6-thiatriazine-1,1-dioxide as claimed in claim 1.

8. A process for the preparation of a 6H-1,2,4,6-thiatriazine-1,1-dioxide as claimed in claim 1, wherein a substituted 6H-1,2,4,6-thiatriazine-1,1-dioxide of the formula

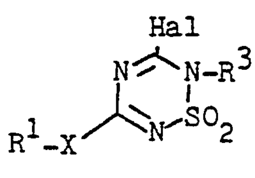

where $R^1$, X and $R^3$ have the meanings given in claim 1 and Hal is halogen, is reacted with a compound of the formula

$$H—Y—R^2$$

where $R^2$ and Y have the meanings given in claim 1, or with an alkali metal salt, alkaline earth metal salt or ammonium salt of this compound, in the presence or absence of an inert organic solvent and in the presence or absence of an acid acceptor.

**Claims** (for the Contracting State AT)

1. A herbicide containing a 6H-1,2,4,6-thiatriazine-1,1-dioxide of the formula

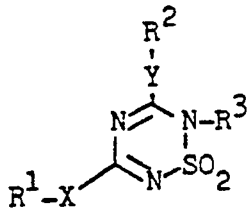

where
$R^1$ is methyl, ethyl, propyl or butyl,
$R^2$ is methyl, ethyl, propyl, butyl, pentyl, hexyl, allyl, methallyl, propargyl, butyn-1-yl, butyn-2-yl, chloroethyl, chloropropyl, chloropropargyl, 4-chlorobut-1-yn-3-yl, chloroallyl, methoxyethyl, ethoxyethyl, 3-methoxypropyl, 3-methoxybutyl, 1-methoxy -but-2-yl, methylmercaptoethyl, ethylmercaptoethyl, 3-methylmercaptopropyl, methylmercaptobutyl, benzyl optionally substituted by halogen, methyl, halomethyl or halomethoxy, or a radical of propionic acid ethyl ester or propanonyl when $R^1$ and $R^3$ are each methyl, or pentenonyl when $R^1$ is ethyl and $R^3$ is methyl, or phenyl optionally substituted by halogen, methyl, ethyl, propyl, butyl, halomethyl, cyano, nitro, methoxy, halomethoxy, methoxycarbamyl, methylureido, methylmercapto, halomethylmercapto, methylsulfinyl, methylsulfonyl, halomethylsulfinyl or halomethylsulfonyl,
$R^3$ is methyl, ethyl or propyl, and
X and Y are oxygen or sulfur.

2. A herbicide as claimed in claim 1, wherein $R^1$ is methyl, $R^2$ is halobenzyl, $R^3$ is methyl, and X and Y are oxygen.

3. A herbicide containing a solid or liquid carrier and a 6H-1,2,4,6-thiatriazine-1,1-dioxide as defined in claim 1.

4. A process for the production of a herbicide, wherein a solid or liquid carrier is mixed with a 6H-1,2,4,6-thiatriazine-1,1-dioxide as defined in claim 1.

5. A process for controlling unwanted plants, wherein the unwanted plants or the soil are treated with a herbicidally effective amount of a 6H-1,2,4,6-thiatriazine-1,1-dioxide as defined in claim 1.

6. A process for the preparation of a 6H-1,2,4,6-thiatriazine-1,1-dioxide as defined in claim 1, wherein a substituted 6H-1,2,4,6-thiatriazine-1,1-dioxide of the formula

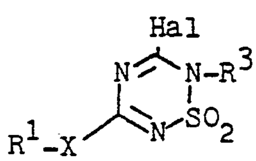

where $R^1$, X and $R^3$ have the meanings given in claim 1 and Hal is halogen, is reacted with a compound of the formula

$$H—Y—R^2$$

where $R^2$ and Y have the meanings given in claim 1, or with an alkali metal salt, alkaline earth metal salt or ammonium salt of this compound, in the presence or absence of an inert organic solvent and in the presence or absence of an acid acceptor.

**Revendications** (pour les Etats contractants : BE, CH, DE, FR, GB, IT, LI, NL, SE)

1. 6H-1,2,4,6-thiatriazin-1,1-dioxyde de formule

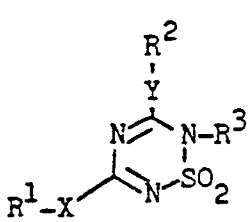

(I)

dans laquelle

$R^1$ représente méthyle, éthyle, propyle, butyle,

$R^2$ méthyle, éthyle, propyle, butyle, pentyle, hexyle, allyle, méthallyle, propargyle, butin-1- ou -2-yle, chloréthyle, chloropropyle, chloropropargyle, 4-chlorobutin-1-yle-3, chlorallyle, méthoxyéthyle, éthoxyéthyle, 3-méthoxypropyle, 3-méthoxybutyle, 1-méthoxy-butyl-2, méthylmercaptoéthyle, éthylmercaptoéthyle, 3-méthyl-mercaptopropyle, méthylmercaptobutyle, reste benzyle, éventuellement substitué par halogène, méthyle, halogénométhyle, halogénométhoxy, reste du propionate d'éthyle ou propanonyle si $R^1$ et $R^3$ = méthyle, pentenonyle, si $R^1$ = éthyl et $R^3$ = méthyle, ou phényle éventuellement substitué par halogène, méthyle, éthyle, propyle, butyle, halogénométhyle, cyano, nitro, méthoxy, halogénométhoxy, méthoxycarbamoyle, méthyluréido, méthylmercapto, halogénométhylmercapto, méthylsulfinyle, méthylsulfonyle, halogénométhylsulfinyle ou halogénométhylsulfonyle,

$R^3$ méthyle, éthyle, propyle et

X et Y oxygène ou soufre.

2. Composé selon la revendication 1, caractérisé par le fait que $R^1$ représente méthyle, $R^2$ halogénobenzyle, $R^3$ méthyle, X et Y oxygène.

3. Herbicide contenant un 6H-1,2,4,6-thiatriazin-1,1-dioxyde selon la revendication 1.

4. Herbicide contenant un composé selon la revendication 2.

5. Herbicide contenant un véhicule solide ou liquide et un 6H-1,2,4,6-thiatriazin-1,1-dioxyde selon la revendication 1.

6. Procédé de préparation d'un herbicide caractérisé par le fait qu'on mélange un véhicule solide et liquide avec un 6H-1,2,4,6-thiatriazin-1,1-dioxyde selon la revendication 1.

7. Procédé de lutte contre les plantes indésirables caractérisé par le fait qu'on traite les plantes indésirables ou le sol avec une quantité suffisante comme herbicide d'un 6H-1,2,4,6-thiatriazin-1,1-dioxyde selon la revendication 1.

8. Procédé de préparation d'un 6H-1,2,4,6-thiatriazin-1,1-dioxyde selon la revendication 1, caractérisé par le fait qu'on fait réagir, éventuellement dans un solvant organique, inerte et éventuellement en présence d'un accepteur d'acide, un 6H-1,2,4,6-thiatriazin-1,1-dioxyde substitué de formule

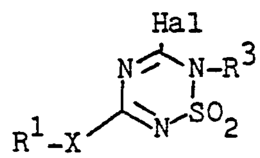

dans laquelle $R^1$, $R^3$ et X ont la signification donnée à la revendication 1 et Hal représente un atome d'halogène, avec un composé de formule

$$H—Y—R^2$$

dans laquelle $R^2$ et Y ont la signification donnée dans la revendication 1 ou leur sel alcalin, alcalino-terreux ou d'ammonium.

**Revendications** (pour l'Etat contractant AT)

1. Herbicide contenant un 6H-1,2,4,6-thiatriazin-1,1-dioxyde de formule

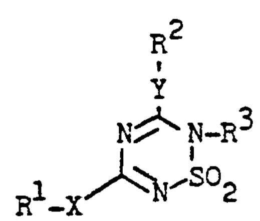

(I)

dans laquelle

$R^1$ représente méthyle, éthyle, propyle, butyle,

$R^2$ méthyle, éthyle, propyle, butyle, pentyle, hexyle, allyle, méthallyle, propargyle, butin-1- ou 2-yle, chloréthyle, chloropropyle, chloropropargyle, 4-chlorobutin-1-yle-3, chlorallyle, méthoxyéthyle, éthoxyéthyle, 3-méthoxypropyle, 3-méthoxybutyle, 1-méthoxy-butyl-2, méthylmercaptoéthyle, éthylmercaptoéthyle, 3-méthylmercaptopropyle, méthylmercaptobutyle, reste benzyle, éventuellement substitué par halogène, méthyle, halogénométhyle, halogéniméthoxy, reste du propionate d'éthyle ou propanonyle si $R^1$ et $R^3$ = méthyle, pentenonyle, si $R^1$ = éthyl et $R^3$ = méthyle, ou phényle éventuellement substitué par

halogène, méthyle, éthyle, propyle, butyle, halogénométhyle, cyano, nitro, méthoxy, halogénométhoxy, méthoxycarbamoyle, méthyluréido, méthylmercapto, halogénométhylmercapto, méthylsulfinyle, méthylsulfonyle, halogénométhylsulfinyle ou halogénométhylsulfonyle,

$R^3$ méthyle, éthyle, propyle, et

X et Y oxygène ou soufre.

2. Herbicide selon la revendication 1, caractérisé par le fait que $R^1$ représente méthyle, $R^2$ halogénobenzyle, $R^3$ méthyle, X et Y oxygène.

3. Herbicide contenant un véhicule solide ou liquide et un 6H-1,2,4,6-thiatriazin-1,1-dioxyde selon la revendication 1.

4. Procédé de préparation d'un herbicide caractérisé par le fait qu'on mélange un véhicule solide et liquide avec un 6H-1,2,4,6-thiatriazin-1,1-dioxyde selon la revendication 1.

5. Procédé de lutte contre les plantes indésirables caractérisé par le fait qu'on traite les plantes indésirables ou le sol avec une quantité suffisante comme herbicide d'un 6H-1,2,4,6-thiatriazin-1,1-dioxyde selon la revendication 1.

6. Procédé de préparation d'un 6H-1,2,4,6-thiatriazin-1,1-dioxyde selon la revendication 1, caractérisé par le fait qu'on fait réagir, éventuellement dans un solvant organique, inerte et éventuellement en présence d'un accepteur d'acide, un 6H-1,2,4,6-thiatriazin-1,1-dioxyde substitué de formule

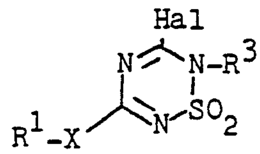

dans laquelle $R^1$, $R^3$ et X ont la signification donnée à la revendication 1 et Hal représente un atome d'halogène, avec un composé de formule

$$H—Y—R^2$$

dans laquelle $R^2$ et Y ont la signification donnée dans la revendication 1 ou leur sel alcalin, alcalino-terreux ou d'ammonium.